# EUROPEAN PATENT APPLICATION

(11) **EP 3 067 783 A1**
(43) Date of publication of application: **14.09.2016**
(21) Application number: 15158556.9
(22) Date of filing: 11.03.2015
(51) Int. Cl.: G06F 3/01, A61B 5/00, G01C 22/00

(54) **METHOD AND SYSTEM TO TRACK HUMAN LOCOMOTION BY RELATIVE POSITIONAL TRACKING OF BODY PARTS OF THE HUMAN**

(71) Applicant: ETH Zurich, 8092 Zurich (CH)
(72) Inventor: Kunz, Andreas, 8044 Zurich (CH); Zank, Markus, 8932 Mettmenstetten (CH); Nescher, Thomas, 9492 Eschen (LI)

(57) **Abstract**

A system (10) to track human locomotion by relative positional tracking of body parts (21, 22) of a user (20) comprises at least two sensors units (11, 12) at each limb (21, 22) as body part for obtaining the relative position of each limb (21, 22) over time to determine the position of the user (20) in space. One sensor at each limb (21, 22) is an electromagnetic sensor detecting signals issued by a central emitter, here integrated in the head unit (13), or localized at a different place at the user (20). The global position of a foot (21, 22) is determined based on its local position, whenever a foot (21 or 22) enters a stance phase, determined by a zero-velocity of that foot (21 or 22).

## Description

### TECHNICAL FIELD

The present invention relates to a method and a system to track human locomotion by relative positional tracking of body parts of the human.

### PRIOR ART

Many applications require tracking a person's movement in real time with high precision for providing multi-modal interactivity or for movement data analysis. Applications of human body tracking systems can be found in education, training, telepresence, medicine, psychology, entertainment, architecture, design, research, and several industrial planning tasks. E.g. to enable human walking in virtual environments, a tracking system tracks a person's viewpoint (i.e. the head) and maps real world movements to movements in the virtual environment (VE). The VE is visualized with the help of a head-mounted display (HMD).

Real walking for navigation in virtual environments (VEs) provides a compelling user experience and was shown to be more presence enhancing than other navigation metaphors inter alia in the document from R. A. Ruddle and S. Lessels. "The benefits of using a walking interface to navigate virtual environments" in ACM Transactions on Computer-Human Interaction, 16(1):1-18, 2009. Such VR systems are typically composed of an HMD to visualize the VE, a tracking system, and a computer with some software to render the VE. The tracking system tracks the user's viewpoint in the real environment and, if required, hands, feet, and other limbs as well. However, to keep the user immersed in the VE, such systems have high requirements in terms of accuracy, latency, range, resolution, etc. This significantly increases the cost and also the installation effort.

The recent availability of low-cost consumer HMDs has greatly reduced the cost for building VR systems. Hence, the major challenge remains designing a low-cost tracking solution which fulfills the requirements for VR applications.

Most of today's tracking systems require setting up a static reference system such as visual markers (fiducials) or cameras in a dedicated area where the tracking is performed (the tracking space). If a person is to be tracked at a new place, the tracking space must first be equipped again with the static reference system and calibrated. Furthermore, these systems do not scale well with the size of the tracking space due to increased cost or setup time. Other tracking systems rely on an existing infrastructure that provides a reference system. E.g. the global positioning system (GPS) enables outdoor tracking by relying on a worldwide reference infrastructure. Other techniques e.g. employ wireless network stations for localization. However, these tracking techniques usually do not provide sufficient accuracy and precision as required by human-computer interaction or VR applications.

Relative tracking is a different approach with the aim to allow for real-time tracking with high precision, low latency, and a high update rate, independent of the size of the tracking space. To achieve this and to avoid expensive external references, relative tracking provides a tracking system where all references are attached to the tracked person. This way, the tracked person carries the system along with himself and the tracking system always operates in a relatively small tracking volume given by the size of the tracked person.

In order to infer the absolute movement of a person, the relative movement of body parts is used. Previously, this has been done with inertial tracking systems attached to a person's feet. For every step, the measured acceleration is integrated twice to obtain the step length and direction which is added to the absolute position. However, inherent measurement errors will also be integrated twice which causes the velocity and position estimation to deteriorate quickly over time. To reduce this problem the integrator has to be reset from time to time. Human locomotion allows for such a recalibration during the foot's stance phase. During the stance phase, the foot is not moving which implies that the velocity estimate of the sensor attached to the foot must be zero. However, other body parts cannot be tracked robustly with pure inertial systems as typically no static reference is available.

Sometimes magnetic sensors are employed for tracking at least the the heading of limbs or the head. E. Hodgson, E. Bachmann, D. Vincent, M. Zmuda, D. Waller, and J. Calusdian. WeaVR: developed within the paper "A self-contained and wearable immersive virtual environment simulation system", published in Behavior Research Methods, pages 1-12, 2014, a VR system based on IMUs and using magnetic sensors to track at least the planar orientation of limbs or the head using earth's magnetic field. However, magnetic orientation tracking based on earth's magnetic field is inaccurate and not suitable for indoor use due to interferences.

E. Foxlin discloses in "Pedestrian tracking with shoe-mounted inertial sensors" in Computer Graphics and Applications, 25(6):38-46, Nov.-Dec. 2005, a system to infer the absolute movement of a person by using the relative movement of body parts with regard to one initial starting point, wherein inertial measurement units (IMUs) are attached to a person's feet. For every step, the measured acceleration is integrated to obtain the step length and direction which is added to the absolute position. However, inertial sensors suffer from drift, which increases quadratically over time.

Two further prior art documents disclose a user-worn electromagnetic position tracker for localizing a walking user. These are the publications by K. Yamanaka, M. Kanbara, and N. Yokoya "Localization of walking or running user with wearable 3d position sensor" in Artificial Reality and Telexistence, 17th International Conference on, pages 39-45, Nov 2007, as well as by A. Hamaguchi, M. Kanbara, and N. Yokoya, "User localization using wearable electromagnetic tracker and orientation sensor" in Wearable Computers, 2006 10th IEEE International Symposium on, pages 55-58, Oct 2006. Instead of using IMUs, a wearable electromagnetic tracker is used for relative tracking.

### SUMMARY OF THE INVENTION

Tracking human movements and locomotion accurately in real time - e.g. for providing real walking in virtual environments - requires expensive tracking systems which need a lot of time to install. Furthermore, the costs typically increase with the size of the tracked space as these systems rely on external references.

It is therefore an aim of the present invention to present a method and a system which significantly reduce costs for tracking human locomotion in large, potentially unlimited tracking spaces.

Furthermore, it is an object of the invention to provide a tracking system allowing for real-time continuous 6-degree-of-freedom (6 DOF) tracking with high precision, low latency, and a high update rate independent of the tracking space's size.

A system according to the inventions, used to track human locomotion by relative positional tracking of body parts of a user comprises at least two sensors attached to the feet for obtaining the relative position of each limb over time to determine the position of the user in space. One sensor at each limb is an electromagnetic sensor detecting signals issued by an emitter localized at a different place on the user. The global position of a sensor or emitter is determined based on its measured local position and the calculated global and measured local positions of another sensor or the emitter.

The invention provides inter alia a method to track human locomotion by relative positional tracking of the feet of the human. Relative tracking avoids expensive external references since, with relative tracking, the tracking system and all references are attached to the tracked person. This way, the system is self-contained and can be deployed rapidly. As the tracking system only has to operate in a person-sized volume, costs are reduced significantly because even low-cost tracking systems can provide a good precision and accuracy for such a small volume.

The features according to the invention employ a low-cost user-worn position and orientation tracking system to track the position and orientation of limbs including the user's feet. To associate the limbs' relative movement within the user's local coordinate system with the absolute movement in a given environment, the feet stance phase is used.

Whenever a foot enters the stance phase, the relative position is locked onto the absolute position and used as a local origin, because the foot is not moving. This allows tracking a person with all locally tracked objects - e.g. limbs, head, etc. - by temporarily locking the absolute position alternately of the user's feet in a step by step manner. This tracking works continuously and in real time without any external references.

In other words, the invention locks the absolute position of the relative sensor with the most certain position/velocity estimate and moves the local coordinate system relative to this one. In an embodiment, an IMU is used to detect the stance phase during human locomotion and choose the corresponding sensor as current origin. During the switch between origins, an error in the position estimate occurs. While this error can also accumulate over time, it only occurs once per step and is not integrated twice over time.

When a foot is standing, the certainty of its position and orientation is maximal and its sensor is therefore used as the local origin. When the foot starts moving and no other sensor is stationary yet, its global position is estimated based on the acceleration and gyroscope data from an IMU. Due to imperfect sensor signals, the uncertainty of the estimate will also increase over time, but the integration time is very short. Once another 6 DOF position sensor has zero velocity or lower position and orientation uncertainty, the local origin is switched to this sensor.

It is noted that it is not necessary to track the feet; actually relative positional tracking of any body part, that allows for zero velocity updates, would also be fine. The method works for any user, which can be a human person but also any animal, device or robot as long as there are phases in which one or more of the sensors have zero velocity.

Further embodiments of the invention are laid down in the dependent claims.

Due to the principle of human walking, one foot always touches the ground (stance phase) while the other leg swings (swing phase). Hence, the local position of a foot in the stance phase can be locked onto the global absolute position and orientation because the foot is not moving. This allows for tracking the absolute position and orientation of all other points as well by associating their relative position/orientation to the fixed foot. Most notably, this technique allows tracking the absolute position/orientation of the other foot while it is moving until it enters the stance phase. At this point, the reference system is moved from the previous foot to the foot that just entered the stance phase. If the standing foot starts moving while no other sensor has zero velocity, a short term position estimation based on the IMU data is used to estimate its global position.

Using this stance phase by stance phase update, the whole locomotion - including any locally tracked object - of a walking person can be tracked in an absolute reference system. The tracking that provides absolute movement data runs continuously in real time and not only on a step by step basis as the switch of the linkage between local and global coordinate system is done transparently. In order to detect the stance phase, existing approaches based on inertial tracking systems can be used.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a user wearing elements of a system according to an embodiment of the invention;
- Fig. 2: shows a block diagram of the components of an embodiment of the system according to Fig. 1;
- Fig. 3: shows a block diagram of the components of the preferred embodiment of the system;
- Fig. 4: shows the lower limbs of a user with feet and in different phases of a movement using a system according to Fig. 2;
- Fig. 5a: shows tracking data of walking along the X-direction and Y-direction with the current base foot; and
- Fig. 5b: shows the tracked X and Y position over time.

### DESCRIPTION OF PREFERRED EMBODIMENTS

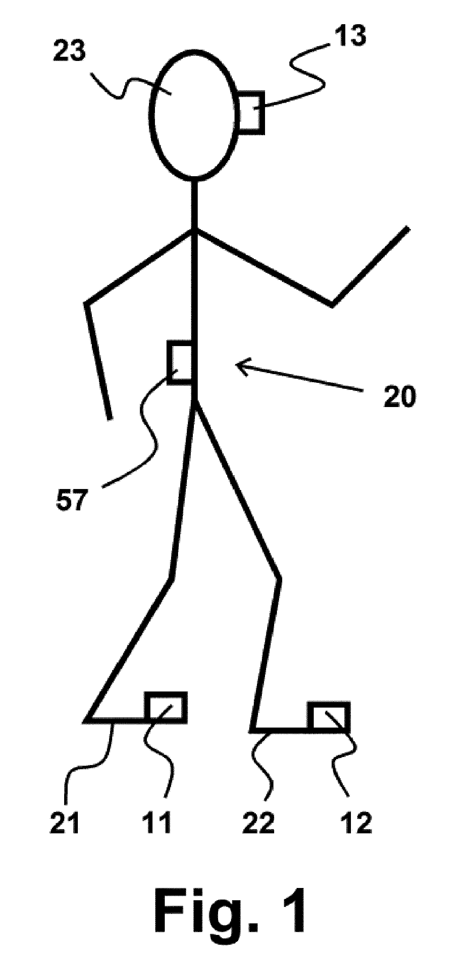

Fig. 1 shows a user 20 wearing elements 11, 12 and 13 of a system according to an embodiment of the invention;

A system 10 according to an embodiment of the invention comprises a hardware setup based on the Razer Hydra electromagnetic trackers 41 and 42 integrated into foot sensor units 11 and 12. The Razer Hydra is a motion and orientation detection game controller developed by Sixense Entertainment Inc. and marketed by Razer. It uses a weak electromagnetic field to detect the position and orientation of the controllers with a precision of 1 mm and 1°; it has six degrees of freedom. Alternative electromagnetic systems are the products by Ascension Technology Corporation or Polhemus.

Also any other measurement system that provides position and orientation measurements between the connected sensors can be used, including but not limited to mechanical connections and goniometers, optical measurement systems (structured light patterns as in Kinect 1, stereo vision, time-of-flight, or the like), and acoustic measurement systems.

In other words, a 6 DOF tracking system is used which is carried along by the user 20. It determines the position and orientation of two 6 DOF sensors 41 and 42 which are included in the sensor units 11 and 12 and attached to the user's feet 21 and 22. The 6 DOF sensors 41 and 42 are preferably positioned on top of the feet tip. The positions of the boxes 11 and 12 reflect this, but the sensor units 11 and 12 comprise, as will be explained below, further sensors preferably attached at the heel of each foot 21 and 22. They can also be attached at the instep of the foot or at any other location that is stationary during the stance phase.

In an embodiment, the head unit 13 comprises an emitter 14 attached to a head-mounted display (HMD) 59. This head mounted display can either overlay additional data over the real environment on a semi-transparent display or video image (Augmented Reality) or replace the real view by a completely artificial image (Virtual Reality). Preferably, the emitter 14 is located attached to the user 20 somewhere on the torso, e.g. the back or the hip. In this case the head unit 13 comprises an HMD 59 and a 6 DOF sensor 43. This allows tracking the user's viewpoint at the head 23with respect to his feet 21, 22 or vice versa

The connection between the units 11, 12, 13, 14 and 57 can be wire-based or wireless.

The control unit 57 is the processor or computer adapted to execute the calculation when determining positions, distances, orientations etc. based on information transmitted from the different sensors to the control unit, by wire or wireless. It is preferred that the base station 14 is not provided within or at one of the sensor units 11 and 12 at the limbs of the user. The control unit 57 can be provided at the head unit 13 but can preferably be provided on the user's torso or at the hand held device providing a display as virtual reality element.

The third sensor 43 can also be provided as a specific visual screen as a tablet or handheld device having a display. Then the user 20 is not immersed as with an HMD but the virtual image shown on the display is simply overlapping the image portion of the real world "behind" the screen. Then instead of the viewpoint of the user 20, the position and orientation of such a device is tracked, but it can still be complemented by an additional sensor for determining the exact viewpoint of the user.

The foot sensor units 11 and 12 have additional Resense IMU 51 and 52 attached to the heel of each feet for detecting the stance phase similar to the setup used in the document of M. Zank, T. Nescher, and A. Kunz in "Robust prediction of auditory step feedback for forward walking" in Proceedings of the 19th ACM Symposium on Virtual Reality Software and Technology, pages 119-122. ACM, Oct. 2013. The Resense IMU mentioned above was disclosed by K. Leuenberger and R. Gassert in "Low-power sensor module for long-term activity monitoring" in Annual International Conference of Engineering in Medicine and Biology Society, pages 2237-2241. IEEE, 2011. Of course any other device capable of measuring 3 axis acceleration and rate of turn can be used instead.

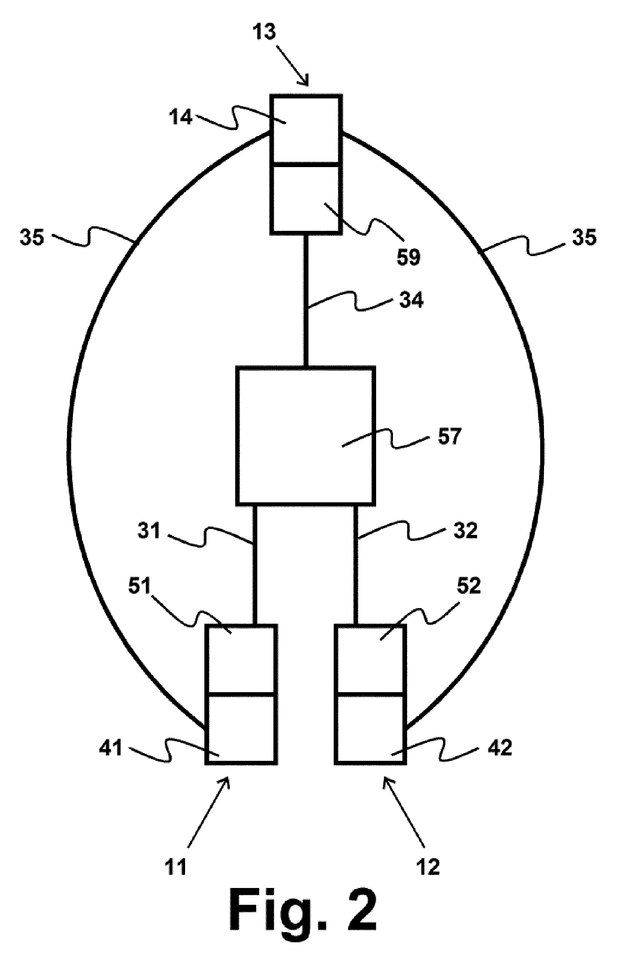

Fig. 2 shows a block diagram of the components of an embodiment of system 10 according to Fig. 1. The system 10 comprises foot sensor units 11 and 12 and a head unit 13. A central emitter 14 is shown in 13. Connection lines 31, 32, 34 and 35 symbolize data transmission lines which can be wire-based or use wireless communication.

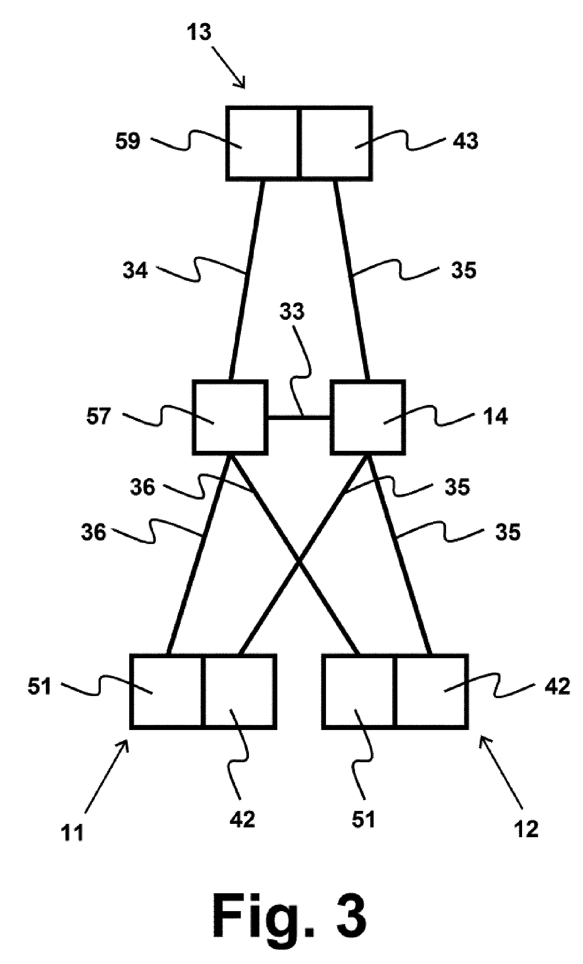

Fig. 3 shows a block diagram of the preferred embodiment of system 10. The system 10 comprises foot sensor units 11 and 12 and a head unit 13, all comprising a 6 DOF sensor (41, 42, 43). A central emitter 14 is located at the user's torso. Connection lines 31, 32, 33, 34 and 35 symbolize data transmission lines which can be -wire-based or use wireless communication.

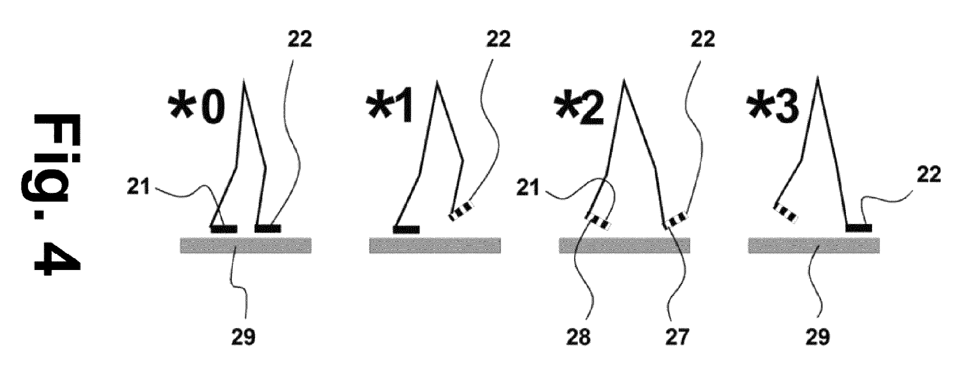

The tracking principle is shown in Fig. 4 in different phases *0 to *3. Fig. 4 shows the lower limbs of a user 20 with feet 21 and 22 in different phases of a movement using a system according to Fig. 2. The user 20 is walking on ground 29.

Initially (phase *0), it can be assumed that a user 20 is standing, and both feet 21 and 22 are fixed (stance phase). Therefore, any foot can be assigned to be the reference foot. This is shown by the thick line representing the foot 21 and 22, respectively.

The central unit 57 links the local coordinate system of the user-worn tracking system with some global reference system. As soon as the user 20 starts walking and lifts one foot 22 (phase *1), the foot 21 that remains fixed (in the stance phase) will be used as the origin for the local tracking system. This is shown by showing the foot 22 in motion by a dashed line. Hence, all other locally tracked points, e.g. the other foot 21 as well as the head 23 with the viewpoint sensor 13, can also be tracked in the absolute reference system.

Next, the reference is switched when the other foot 22 starts touching the floor in phase *3. However, the stance phases of both feet might not overlap. It can occur that the reference foot's 21 heel 28 is lifted while the non-reference foot 22 is still moving. Hence, for a short time interval both feet are not fixed. This period of time is longer when the user 20 is running.

In order to guarantee tracking during this period, the global position and orientation of the current reference foot 21 is estimated based on data from IMU 51. Foot 21 is still used as the reference for the local system.

Finally (phase *3), as soon as the non-reference foot 22 enters the stance phase, the reference is switched as shown by the bold line for this foot 22 and the tracking continues as in phase *1.

Using this stance phase (between *1) by stance phase (to *3) update, the locomotion - including any locally tracked point - of a walking person can be tracked in an absolute reference system. The tracking that provides absolute movement data runs continuously in real time and not only on a step by step basis as the base switch is done transparently.

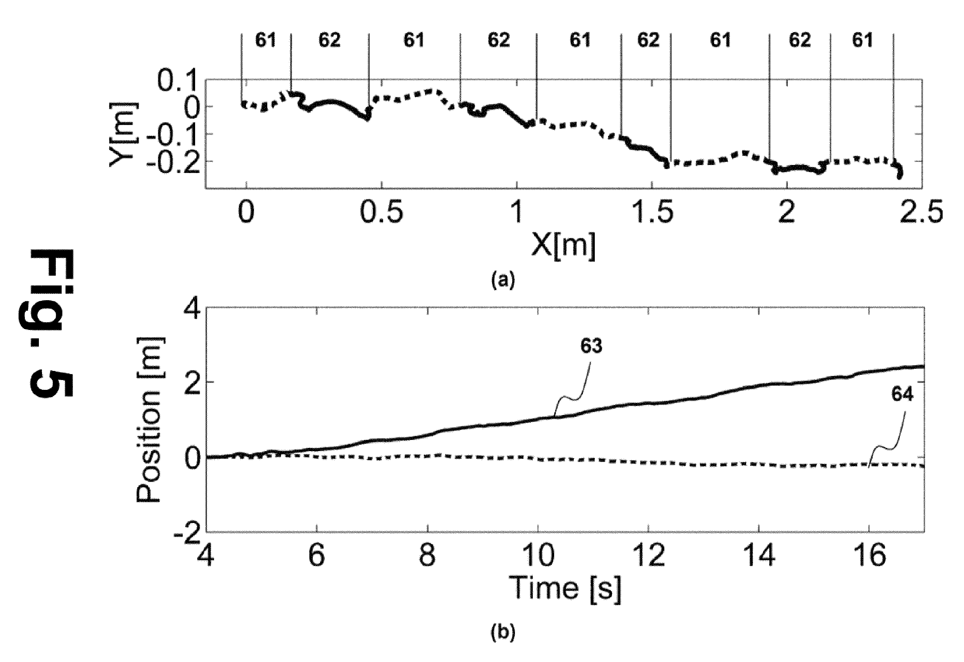

Fig. 5 shows the results of the application of the system 10. Fig. 5a shows tracking data of walking where the X-Y-plane describes the ground plane and Fig. 5b shows the tracking position over time.

The different periods when one foot 21 as well as the other foot 22 is used as local origin are shown as distance portions 61 and 62, respectively.

Fig. 5b shows with graph 63 the movement of user 20 in X-direction and graph 64 shows the movement in Y-direction over time in seconds. The system 10 is capable of correctly tracking a moving user 20. While the self-carried electromagnetic position tracker itself is drift-free, the presented tracking approach still accumulate errors over time as for all relative tracking approaches, i.e. the accuracy of the absolute position estimate degrades. However, this error only occurs at the base switch and very short estimation periods during phase *2 and is bounded by the measurement error of the position tracker. Thus, it does not increase quadratically with the total time as IMU tracking systems do. Hence, the error is not critical for virtual reality or human computer interaction applications as high precision and low latency is most important but not accuracy.

The approach solves the problem of relative tracking and can robustly track the absolute movement of human locomotion in real time with high precision, high accuracy and high update rate suitable for various applications. Furthermore, it can not only track the feet but also the full position and orientation (all 6 DOF) of arbitrary body parts (limbs, head, etc.) or of an external especially hand-held screen, which is then to some extent an extension of the body. This allows tracking a person's viewpoint e.g. for virtual reality applications like real walking in virtual environments and even allows for full virtual embodiment.

**LIST OF REFERENCE SIGNS**

| | | | |
|---|---|---|---|
| 10 | system | 34 | transmission line |
| 11 | foot sensor unit | 35 | transmission line |
| 12 | foot sensor unit | 36 | transmission line |
| 13 | head unit | 41 | 6 DOF sensor |
| 14 | central emitter | 42 | 6 DOF sensor |
| 20 | user | 43 | 6 DOF sensor |
| 21 | foot as body part / part of | 51 | IMU sensor |
| | limb | 52 | IMU sensor |
| 22 | foot as body part / part of | 57 | central unit |
| | limb | 59 | display, especially HMD |
| 23 | head | 61 | distance traveled with foot 21 |
| 27 | heel | | as origin |
| 28 | heel | 62 | distance traveled with foot |
| 29 | ground | | 22 as origin |
| 31 | transmission line | 63 | movement in x-direction |
| 32 | transmission line | 64 | movement in y-direction |
| 33 | transmission line | | |

## Claims

1. System (10) to track human locomotion by relative positional tracking of body parts (21, 22) of a user (20), comprising at least two sensors (11, 12; 41, 42, 51, 52) at limbs (21, 22) as body parts, especially the feet of the user, for obtaining the relative position of each limb (21, 22) over time to determine the position of the user (20) in space (29) and a base station (14) localized at a different place at the user (20), wherein one sensor (41, 42) at each limb (21, 22) is an 6 DOF position sensor (41, 42) detecting position and orientation relative to the base station (14), wherein the global position and orientation of a moving sensor or emitter (41, 42, 14) are continuously determined based on its local position and the global and local positions and orientations of a stationary foot (22, 21), whenever a foot (21 or 22) enters a stance phase (*1 or *3), determined by a zero-velocity of that foot (21 or 22), its global position is assumed to be constant.

2. System according to claim 1, wherein the sensors further comprise inertial measurement units (51, 52) at each limb, wherein additional movement of one of the 6 DOF position sensors (41, 42) in a movement phase (*2) between two stance phases (*1 and *3) is estimated using data from the IMUs (51, 52).

3. System according to claim 1, further comprising a database with data of virtual reality objects in a predetermined virtual reality space; wherein, at a starting point in time, the position of a not-moving (*1) user (20) in the virtual reality space is determined, further comprising a display (59) showing the image of the virtual reality at the virtual position of the user (20) in the view direction of the user (20), determined by an additional 6 DOF sensor (43) provided to determine the viewing direction of the user (20).

4. System according to claim 3, wherein the 6 DOF sensor (43) is provided at the head (23) of the user (20) to determine its viewing direction on a screen (59), preferably a head mounted display.

5. System according to claim 3, wherein the 6 DOF sensor (43) is provided at a user-associated display (59), wherein the user-associated display (59) can be a hand-held screen or a screen mounted in a predetermined spaced relationship with the user as with an external frame.

6. A method to track human locomotion by relative positional tracking of body parts (21, 22) of a user (20), comprising the steps of
- providing at least one 6 DOF sensor (41, 42) at two limbs (21, 22) as body parts, especially the feet of the user,
- providing a central emitter (14) localized at a different place on the user (20) than said sensors at the limbs,
- obtaining the relative position of each limb (21, 22) over time to determine the position of the user (20) in space detecting position and orientation relative to the central emitter (14), and
- determining the global position of a foot (21, 22) based on its local position, whenever a foot (21 or 22) enters a stance phase (*1 or *3), determined by a zero-velocity of that foot (21 or 22).

7. The method according to claim 6, wherein the base station is adapted to detect additional movement of one of the 6 DOF sensors (41, 42) in a movement phase (*2) between two stance phases (*1 and *3) and the method further comprises:
- providing an inertial measuring unit (51, 52) at each limb, and
- estimating the additional movement using data from the IMUs (51, 52).

8. The method according to claim 6 or 7, further comprising
- providing a database with data of virtual reality objects in a predetermined virtual reality space,
- providing a display (59) adapted to show the image of the virtual reality from the database,
- providing an additional 6 DOF sensor (43) adapted to detect information relating to the viewing direction of the user,
- determining, at a starting point in time, the position of a not-moving (*1) user (20) in the virtual reality space,
- determining through data acquired by the additional 6 DOF sensor (43) the viewing direction of the user (20), and
- showing on the display (59) the image of the virtual reality at the virtual position of the user (20) in the view direction of the user (20) as determined.
